(19) **Europäisches Patentamt**

**European Patent Office**

**Office européen des brevets**

(11) Publication number: **0 176 337**
**B1**

(12) **EUROPEAN PATENT SPECIFICATION**

(45) Date of publication of patent specification: **29.11.89**

(21) Application number: **85306754.4**

(22) Date of filing: **23.09.85**

(60) Divisional application **88112710 filed on 04.08.88.**

(51) Int. Cl.⁴: **A 61 F 2/24**

(54) **Heart valve prosthesis.**

(30) Priority: **24.09.84 US 653960**

(43) Date of publication of application:
**02.04.86 Bulletin 86/14**

(45) Publication of the grant of the patent:
**29.11.89 Bulletin 89/48**

(84) Designated Contracting States:
**AT BE CH DE FR GB IT LI NL SE**

(56) References cited:
**EP-A-0 023 797**
**US-A-4 240 161**
**US-A-4 306 319**
**US-A-4 326 304**
**US-A-4 373 216**
**US-A-4 451 937**

(73) Proprietor: **Carbomedics Inc.**
**1300 East Anderson Lane**
**Austin Texas 78752 (US)**

(72) Inventor: **Bokros, Jack Chester**
**2204 Manana Street**
**Austin Texas 78732 (US)**

(74) Representative: **Bizley, Richard Edward et al**
**BOULT, WADE & TENNANT 27 Furnival Street**
**London EC4A 1PQ (GB)**

Courier Press, Leamington Spa, England.

## Description

This invention relates to heart valve prostheses for replacement of defective natural valves and more particularly to heart valve prostheses which employ one or more occluders in the form of flat platelike members, although certain features of the invention are applicable to valves having curved occluders.

Various types of heart valve prostheses have been developed which operate hemodynamically as a result of the pumping action of the heart and which are in essence functioning as check valves. Early heart valves employed a ball-and-cage arrangement whereas later versions of heart prostheses have employed one or more occluders generally in the form of a plate or disc which might be flat or of a curved shape. Bokros Patent No. 3,546,711 shows a heart valve having a circular occluder which is pivoted from a hinge pin that coacts with a pair of upstanding fins located on the downstream surface of the occluder. Bokros Patent No. 4,178,639 shows a bi-leaflet heart valve having a pair of platelike members each of which has ears extending from its opposite lateral edges that pivot in spheroidal guides located in the orifice ring. U.S. Patent No. 3,445,863 shows a heart valve arrangement having one or more occluders in the form of flat plates having cut-outs at their edges which are proportioned to interfit with complementary cut-outs in a base ring formed of generally similar material. U.S. Patent No. 4,225,980 shows a metallic heart valve which includes an oval-shaped occluder having cut-outs in opposite lateral edges which coact with parabolic pivots or pegs that extend radially inward from the interior surface of the orifice ring. U.S. Patent No. 4,078,268 shows a bi-leaflet valve having a pair of semicircular occluders which have ears extending upward from the downstream surface thereof that co-act with fulcrums to effect a pivoting action. U.S. Patent No. 4,159,543 shows a variety of bi-leaflet valves, one version of which has grooves cut into the edges of the semicircular occluders at an oblique angle to the surface thereof, which grooves receive generally conical pivot pins that extend radially inward from the interior surface of the orifice ring. U.S. Patent No. 4,373,216 illustrates heart valves having one or more occluders that have aligned notch means in opposite edges of the periphery thereof which coact with elongated aligned protuberances extending inward from the surface of the orifice ring, these protuberances having arcuate opposite lateral surfaces, and which are proportioned to be received within the notch means, and provide tracks for guiding pivotal and translational movement of the occluders.

As is apparent from the foregoing, a wide variety of different heart valves have been designed, and work continues on new heart valve designs in order to still further improve the functioning of these prostheses which are being used in greater quantity each year as surgical techniques improve throughout the world.

The invention provides a heart valve prosthesis comprising a generally annular body which has an interior surface that defines a central passageway for blood flow therethrough, occluder means supported on said body which block the flow of blood in one direction but allow the flow of blood through said passageway in the other direction, said occluder means being formed with aligned notch means at opposite locations in the periphery thereof, a pair of aligned projections projecting generally radially inward from said interior surface of said body, which projections have arcuate opposite lateral surfaces and are proportioned to be received within said notch means, and stop means projecting inward from said interior surface at locations generally flanking each of said projections, characterized in that said projections are pivot posts for said occluder means and that said stop means extend radially farther inward than said pivot posts, said stop means together presenting a pair of curved surfaces in regions adjacent to said pivot posts, each of which curved surfaces lies in a region located intermediate of said opposite arcuate lateral surfaces of said pivot posts and radially inward thereof so as to provide a pair of oppositely disposed fulcrums that alternately assist in defining the opening and closing movement of said occluder means.

Preferably, the pivot posts and the stop means are formed as an integral structure so as to avoid any gap therebetween which would provide a stagnant region where blood clotting might occur.

Brief description of the drawings

Figure 1 is a perspective view of a bi-leaflet heart valve embodying various features of the invention, shown in the open position.

Figure 2 is an enlarged sectional view taken generally along the line 2—2 of Figure 1.

Figure 3 is a fragmentary view similar to Figure 2 showing the leaflets in the closed position.

Figure 4 is a fragmentary view, similar to Figures 2 and 3, of the orifice ring with the leaflets being shown in broken lines in an approximate position they might assume during opening movement.

Figure 5 is a fragmentary plan view of the orifice ring shown in Figure 4.

Figure 6 is a fragmentary sectional view taken along line 6—6 of Figure 5.

Figure 7 is a side view of one of the occluder leaflets with portions broken away to illustrate its interior structure.

Figure 8 is a fragmentary sectional view taken generally along the line 8—8 of Figure 2.

Figure 9 is a perspective view of an alternative embodiment of an annular body suitable for use as a part of a single-occluder heart valve.

Figure 10 is a fragmentary plan view of the annular body shown in Figure 9.

Figure 11 is a perspective view of the heart valve showing the annular body of Figure 9 with the occluder installed and in the open position.

Figure 12 is a side view of the occluder with a portion broken away to show its internal construction.

Figure 13 is a fragmentary front view of the occluder.

Figure 14 is a sectional view of the heart valve shown in Figure 11 depicting in full lines the occluder in an intermediate position as it is moving to the closed position and with the occluder shown twice in broken lines in the position which it assumes in the open position and in the closed position.

Figure 15 is an enlarged fragmentary view of the interior surface of the annular ring emphasizing the pivot support area.

Detailed description of the preferred embodiments

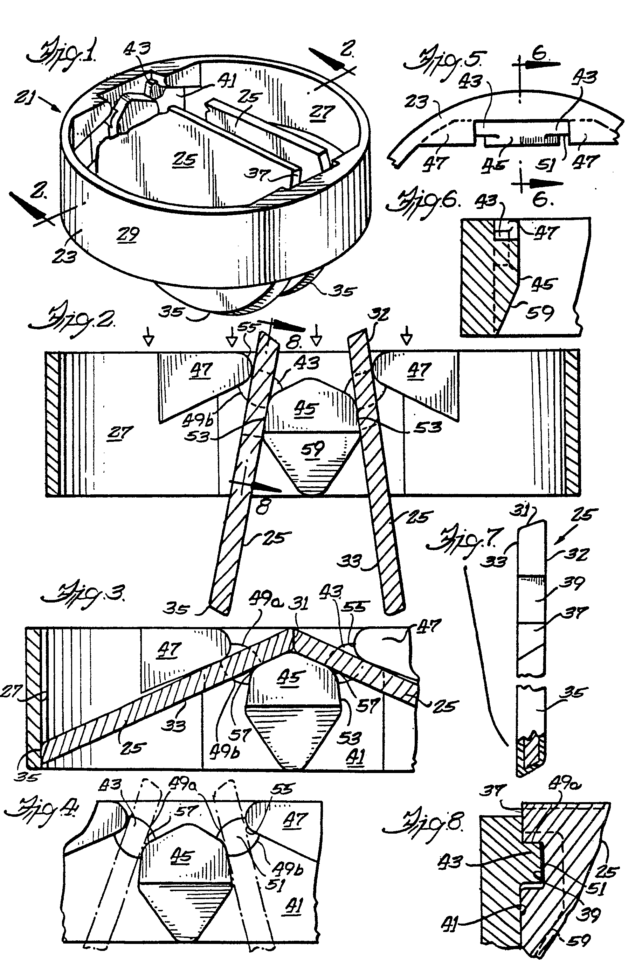

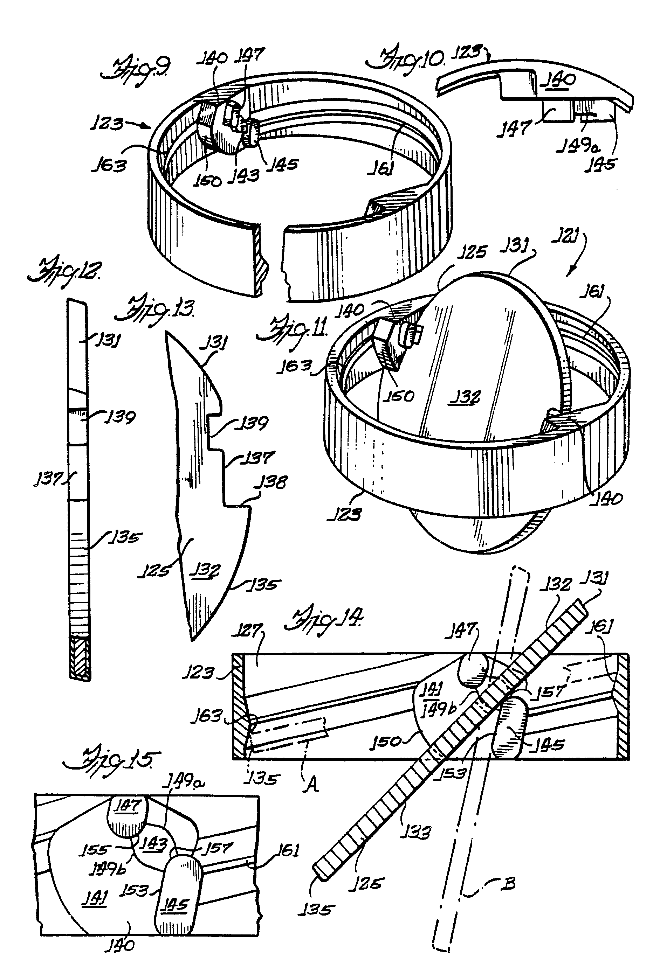

Illustrated in Fig. 1 is a heart valve 21 which consists of an annular valve body or housing 23 which supports a pair of pivoting leaflets or occluders 25 that open and close to control the blood flow through a central passageway or orifice which is defined by the interior surface 27 of the valve body. Inasmuch as the annular body 23 defines the central passageway or orifice, it is sometimes referred to as an orifice-ring. The normal flow of blood through the heart valve 21 is downward in the orientation in which the valve is shown in Figs. 1 and 2, as represented by the small arrows which appear at the top of Fig. 2. It should, of course, be understood that the valve 21 can operate in any orientation and is not significantly affected by gravity. Thus, the terms such as upward and downward, as used hereinafter, are merely employed to facilitate explanation and understanding and are not meant to place any limitations upon the operation of the heart valves being described.

As depicted, the annular body 23 has a smooth exterior surface 29 which is that of the lateral surface of a right circular cylinder. It should be understood that suitable means, such as a peripheral groove or a pair of flats, would usually be provided for attaching a suturing ring to the annular body to facilitate sewing or suturing of the heart valve 21 to the heart tissue. However, inasmuch as the suturing ring and its means of attachment to the heart valve 21 form no part of the present invention, it is simply omitted so as to facilitate the illustration of the specific components of the heart valve with which the invention is concerned. U.S. Patent No. 4,233,690, issued November 18, 1980, illustrates one method of attaching a suturing ring to an annular heart valve body.

The valve body 23 and the leaflets 25 may be made of any suitable material that is biocompatible and nonthrombogenic and that will take the wear to which it will be subjected during countless opening and closing movements of the leaflets. Preferably, the leaflets are made of isotropic graphite, such as that sold under the tradename POCO and that preferably contains between about 5 and about 20 weight percent tungsten to render it radio-opaque and allow x-ray visualization, which graphite has been suitably coated with pyrolytic carbon, such as that sold under the trademark PYROLITE. The lower portion of Fig. 7 shows the pyrocarbon coating on the graphite substrate. Such pyrocarbon gives excellent blood and tissue compatibility and wear-resistance.

The orifice ring 23 can be made in the same fashion using a pyrocarbon-coated substrate, or it can be, and preferably is, made from solid PYROLITE pyrocarbon. A preferred process for forming an orifice ring from all pyrocarbon by coating a mandrel, which is subsequently removed, is disclosed in European Patent Application EP—A—0 055 406.

The illustrated leaflets 25 are flat and have a uniform thickness throughout. Each leaflet 25 has a straight-edge portion 31, which is best seen in Figure 7 and which is oriented at an angle to the downstream surface 33 of the leaflet so that the two straight-edge portions 31 abut in substantially face-to-face contact when the pair of leaflets are in the closed position as depicted in Fig. 3. Generally, the angle will be between about 110° and about 130°, being of course dependent upon the orientation of the leaflets to the horizontal or transverse plane in the closed position, as depicted in Fig. 3. In addition, each of the leaflets 25 has a major arcuate, nearly semicircular, edge 35 (see Fig. 1) and a pair of intermediate or transitional straight edge portions 37 which are flat and perpendicular to the upstream and downstream surfaces of the leaflets. In addition, each leaflet 25 contains a pair of notches 39 disposed in its opposite lateral edges in the regions of the straight edge surfaces 37. As best seen in Fig. 8, the notches are generally rectangular in shape, and all of the defining edges are rounded as opposed to being sharp.

The passageway-defining interior surface 27 of the annular body 23 also has a right circular cylindrical shape for a major portion of its length but is interrupted by a pair of diametrically opposed flat sections 41 from which the support means for holding and defining the movement of the leaflets 25 radially project. The support means includes a pair of pivot posts 43, which are sized to fit within the notches 39 and which pivot posts are flanked by stop means 45, 47. As can be seen from Figs. 1 and 5, the flanking stop means 45, 47 project radially farther inward than do the pivot posts 43. Preferably, the pivot posts 43 and the flanking stop means are formed integrally with one another, and most preferably this overall support structure is formed as an integral part of the annular body 23, as by machining a preshaped body of pyrolytic carbon to final dimensions. As best seen in Fig. 4, the pivot posts 43 each have a pair of opposite arcuate surfaces, a generally upper surface 49a and a generally lower surface 49b, which are referred to as the lateral surfaces of the posts 43, so as to be distinguished from the flat end surface 51 of each pivot post.

The leaflets 25 are suitably assembled with the annular ring 23 by suitably distorting the ring, as by squeezing the ring inward at its relatively thin, hollow, cylindrical regions so that the diametrically opposite, thicker regions where the flat surfaces 41 are located move away from each other sufficiently far to allow the insertion of the notched leaflets. Instead of squeezing, the thicker portions may be pulled outward. An all PYROLITE pyrocarbon annular body 23 has sufficient resiliently to permit this distension and to return to its originally machined configuration. In the assembled heart valve, the depth of the notch 39 is slightly greater than the length of the pivot post 43 so that one of the lateral edge surfaces 37 of the leaflet will bear against a facing flat section 41 of the annular body, as illustrated in Fig. 8.

In the open position depicted in Fig. 2, the leaflets 25 lie at an angle of about 5° to 15° to the centerline of the valve passageway with the upper edge surface of the notch 39 in contact with the arcuate surface 49a of the pivot post 43, see also Fig. 8. The orientation of the leaflet is maintained and defined generally by contact of the downstream surface 33 of the leaflet against side surfaces 53 formed on the central stop 45. In addition, there will be line contact between the upstream surface 32 of the leaflets 25 and an arcuate surface 55 which is formed on the stops 47 in a location that is intermediate of the upstream surface 49a and the downstream surface 49b of the pivot post 43 and is spaced radially inward thereof.

When the downstream (downward as depicted in Fig. 2) flow of blood is discontinued as the contraction of the respective ventricle terminates with respect to an aortic valve, the respective ventricle will then begin to relax in order to draw more blood into the chamber from the atrium, and as a result the back pressure which is present within the aorta causes blood to tend to flow upstream (upward, as illustrated in Figures 2 and 3) causing the leaflets 25 to swing or pivot toward the closed position. The leaflets are quickly displaced slightly upward so that the lower surfaces of the rectangular notches 39 bear against the arcuate surfaces 49b of the pivot posts, and the swinging of the leaflets is guided by contact at these points, together with contact between the upstream surface 32 of the leaflets 25 and the arcuate surface 55 on the stops 47.

When the closed position is reached as depicted in Fig. 3, the arcuate edges 35 of the leaflets 25 lie in contact with the interior surface 27 of the annular body. Accordingly, the shape of the arcuate edge 35 will generally be that of a section of an ellipse, i.e., the intersection between a plane and a right circular cylinder, and the edge is preferably bevelled as shown (Fig. 7) so as to more closely seat against the interior cylindrical surface. In addition, there may be line contact between the arcuate surface 55 of the stops 47 and the upstream surface 32 of the leaflets, and the straight-edge portions 31 of the two leaflets 25 will also abut in substantially face-to-face contact, all as shown in Fig. 3. There possibly may also be some slight touching between the downstream surface 33 of the leaflets 25 and region of a pair of curved surfaces 57 which are formed on the central stop 45 at locations just above the side surfaces 53 and which thus lie intermediate of the upstream and downstream arcuate surfaces 49a, b, and radially inward of the pivot posts.

When the pumping stroke again occurs, the pressure against the upstream surface of the leaflets 25 immediately displaces the leaflets downward against the central stop 45, and pivoting begins guided by the line contact between the undersurface 33 of the leaflets 25 and the curved surfaces 57, as depicted in ghost outline in Fig. 4. In addition, the leaflets will be displaced slightly so that there is contact between the upper surfaces of the notches 39 and the arcuate surfaces 49a on the pivot posts.

By forming the posts 43 and stop means 45, 47 integral with one another, the regions of joinder can be blended together arcuately so there are no sharp depressions or valleys, i.e. no regions of concave curvature having a radius of curvature less than about 0.2 mm., which would be locations ripe for the beginning of clotting; thus, there is a substantial advantage gained from being able to have such a smooth transition from surface-to-surface, particularly where the very ends of the arcuate surfaces are not necessary for guidance. Moreover, the desired smooth swinging of the leaflets between the open and closed positions is further facilitated by the radius of curvature which appears on the surfaces 49a and 49b of the pivot posts 43. In this respect, the focal point of the radius of curvature of each of these surfaces 49a, 49b should lie beyond the center point of the pivot post and may even lie past the opposite side of the pivot post. By examining the leaflets in the closed position as shown in Fig. 3, it will be seen that this arrangement between the arcuate surface 49a and the substantially flat surfaces defining the upper end of the notch 39 creates an immediate sharp pivoting movement which tends to more quickly open the valve in response to the beginning of the pumping stroke. In addition to blending the transition areas between adjacent surfaces, streamlining is also provided as by creating an oblique undersurface 59 on the central stop 45.

In summary, the integral pivot post and stop arrangement minimizes interference with blood flow, while at the same time providing for effective washing of the surfaces to avoid clotting; moreover, very effective and positive control of the swinging of the leaflets is provided as a result of the short pivot posts 43 which are flanked by the stop means which protrude farther radially inward into the passageway and thereby serve not only as stop surfaces, but also fulcrums to assist the pivoting movement. As best seen perhaps by examining Figs. 4 and 8 together, the movement of the leaflet about the surface 49a as a fulcrum is primarily a rolling action, with a minimum of sliding movement, which is important to reduce wear in a device such as this that must continue to operate satisfactorily for an indeterminate number of years. The ability to blend the pivot posts into the stop means, which results from the integral nature of them, allows the design to take advantage of the contact between the pivot post and the stop means which provides overall strengthening of the structure without having to suffer the disadvantage of precisely matching individually machined components to try to avoid minor crevices and valleys that might likely promote the formation of blood clotting.

Depicted in Figs. 9—15 of the drawings is an alternative embodiment of a heart valve 121 which includes an annular body 123 designed to operate with a singular flat occluder or disc 125. Generally, the same principles of design are executed in the heart valve 121 as were hereinbefore explained in detail with respect to the bi-leaflet valve 21, and therefore similar numbers in the 100 series are utilized to refer to comparable components. Moreover, it should be understood that, unless specifically stated hereinafter, the function and construction of the comparable components will be essentially the same as previously described.

The annular body 123 has an interior surface 127 which defines the passage or orifice through which the bloodstream will flow and is illustrated with a smooth exterior cylindrical surface 129 for the reason set forth hereinbefore. The interior surface 127 does differ from that of the bi-leaflet version in that, as described in detail hereinafter, inwardly extending seats 161, 163 are provided against which the arcuate edges of the disc occluder 125 can positively seat.

The occluder 125 generally has the form of a flat disc having an upstream surface 132 and a downstream surface 133. The periphery of the disc includes a minor arcuate section 131, a major arcuate section 135 and a pair of oppositely disposed, straight, parallel intermediate sections 137. Notches 139 of generally rectangular shape are located in the regions of the straight edge portions 137. The shape of the major arcuate edge 135 is generally that of a section of an ellipse, and the shape of the minor arcuate edge section 131 is also generally that of a section of an ellipse with a slightly shorter minor axis. As best seen perhaps in Fig. 13, the intermediate edge portions are each inwardly offset from the outermost lateral extension of the major edge surface by short perpendicluar transitional surfaces 138 that are colinear. As best seen in Fig. 12, the circular disc is also preferably made of isotropic graphite which has been coated with PYROLITE pyrolytic carbon.

The pivot and stop means arrangement is also preferably formed integrally as a part of the annular body 123 for the reasons as hereinbefore described with regard to the annular body 23 illustrated in Figs. 1 through 8; however, the design of the arrangement differs somewhat because of the single occluder concept. Instead of forming a pair of opposed flat regions at opposite locations within an otherwise annular body having an interior passageway of generally circular cross section, improved guidance of the occluder is obtained by providing a pair of protrusions or lands 140 which extend from the inner surface of the annular body 123 and present a pair of oppositely disposed flat surfaces 141. The protrusions 140 lie substantially to the right of a diameter of the passageway which is parallel to the pivot axis as viewed in Fig. 14.

Projecting radially inwardly from each of these flat surfaces 141 is a pivot post 143 and a pair of stops 145, 147. The pivot posts 143 have arcuate, generally upper and lower surfaces 149a, b which are similarly formed with the focal point of the radius of curvature lying beyond the center point of the pivot post and, if desired, past the opposite surface of the post, for the purpose described hereinbefore.

The annular body 123 is suitably distended, as described hereinbefore, so as to allow the occluder 125 to be snapped into place with the notches 139 fitting about the pivot posts 143. As described previously, the length of the pivot post 143 is just less than the depth of the notches 139 so that the flat edge surfaces 137 of the occluder will bear against the flat surface 141 of the protrusions and provide a bearing surface during pivoting between open and closed positions. In addition, each protrusion 140 is formed with a curved undersurface 150 against which the transitional surface 138 wipes during pivoting action.

In the open position, as shown in Figure 11 and in broken lines in Figure 14, the occluder is oriented at between about 5° and 15° to the centerline of the passageway, e.g., at about 10°, lying generally against an inward-facing surface 153 of the stop 145 and with the upper surface of the notch 139 in contact with the upper surface 149a of the pivot post. There may also be contact between the upstream surface 132 of the occluder and the upper stop member 147.

As soon as the pumping stroke of the ventricle ceases for a valve in the aortic position, the back pressure flow lifts the occluder 125 so that the lower surface of the notch 139 contacts the arcuate surface 149b of the pivot post and the upstream surface 132 contacts a curved surface 155 formed on the stop 147 at a location intermediate of the upstream and downstrean surfaces 149a, b of the pivot post 143, both of which surfaces 149b guide the pivoting motion. As pivoting movement continues to an intermediate position, the transitional edge surface 138 of the occluder essentially wipes along the curved undersurface 150 of the protrusion. When the occluder 125 reaches the fully closed position (see broken line illustration "A" in Fig. 14), its major and minor arcuate edges are in contact with the pair of generally semi-elliptical seats 161, 163 which are formed as a part of the annular body 123 projecting inward from its interior surface. Because each of these seats is shaped with a generally conical or oblique configuration, the occluder will be self-centering so long as there is clearance at the notches 139, and there will be line contact between each edge of the occluder and one of the seat surfaces, thus providing a more positive seal around the periphery of the occluder when the heart valve is in the closed position.

This unique self-centering seating feature is applicable to other heart valve designs where a pair of opposed seats can be provided, each of which has a surface that is a portion of generally the surface of a frustum of a cone, one being conical upward and the other being conical downward.

When the ventricle again begins to contract to resume the next pumping stroke, the pressure against the upstream surface 132 causes pivoting to begin, and the flow of blood displaces the occluder 125 slightly downstream so that the upper edge of the notch is in contact with the upper arcuate surface 149a of the pivot post which, along with a curved upper surface 157 formed on the lower stop 145 (also intermediate of the upstream and downstream surfaces 149a and 149b), defines the path of pivoting movement from the closed to the open position. An intermediate position is illustrated in full lines in Fig. 14, and the pivoting movement is terminated when the undersurface 133 of the occluder comes into contact with the stop surface 153 in the fully open position, as shown in broken line outline "B" in Fig. 14. In this position, there is excellent blood flow through the heart valve central passageway because, as best seen in Fig. 11, the post and stop arrangement only extends minimally into the passageway region and because the additional vacant area adjacent the surface 150 of the protrusion further decreases the resistance to flow through the valve.

As in the case of the bi-leaflet valve 21, the streamlined configuration provided by the integral post and stop means, as best seen perhaps in Figure 10, provides a minimum of disruption to flow through the central passageway. Moreover, the integral construction allows one curved surface to be blended into the adjacent curved surface thus avoiding the creation of crevices and/or valleys that tend to permit stagnation and promote blood clotting.

Although the invention has been described with regard to two preferred embodiments, it should be understood that various changes and modification as would be obvious to one having the ordinary skill in this art may be made without departing from the scope of the invention which is defined by the claims appended hereto. For example, with respect to many of the novel features of the invention, the occluders need not be flat but could be curved in cross-section as have been shown in a number of prior art heart valve designs.

## Claims

1. A heart valve prosthesis (21, 121) comprising a generally annular body (23, 123) which has an interior surface (27, 127) that defines a central passageway for blood flow therethrough, occluder means (25, 125) supported on said body which block the flow of blood in one direction but allow the flow of blood through said passageway in the other direction, said occluder means (25, 125) being formed with aligned notch means (39, 139) at opposite locations in the periphery thereof, a pair of aligned projections (43, 143) projecting generally radially inward from said interior surface of said body, which projections have arcuate opposite lateral surfaces and are proportioned to be received within said notch means, and stop means (45, 47, 145, 147) projecting inward from said interior surface at locations generally flanking each of said projections, characterized in that said projections (43, 143) are pivot posts for said occluder means (25, 125) and that said stop means (45, 47, 145, 147) extend radially farther inward than said pivot posts (43, 143), said stop means (45, 47, 145, 147) together presenting a pair of curved surfaces (55, 57, 155, 157) in regions adjacent to said pivot posts (43, 143), each of which curved surfaces lies in a region located intermediate of said opposite arcuate lateral surfaces (49a, 49b) of said pivot posts (43, 143) and radially inward thereof so as to provide a pair of oppositely disposed fulcrums that alternately assist in defining the opening and closing movement of said occluder means (25, 125).

2. A prosthesis as claimed in Claim 1 wherein said stop means (45, 47, 145, 147) is formed integrally with said pivot posts (43, 143).

3. A prosthesis as claimed in Claim 2 wherein the curved surfaces of said stop means (45, 47, 145, 147) are blended into said pivot post arcuate lateral surfaces (49a, 49b).

4. A prosthesis as claimed in Claim 3 wherein said stop means (45, 47, 145, 147) and pivot posts (43, 143) are integral with said annular body (23, 123).

5. A prosthesis as claimed in any one of claims 1 to 4 wherein the focal point of each of said arcuate surfaces (49a, 49b, 149a, 149b) of said pivot posts (43, 143) is located past the center point of said pivot posts (43, 143).

6. A prosthesis as claimed in any one of claims 1 to 5 wherein said occluder means (25, 125) is a single disc (125) having a pair of opposite straight peripheral segments (137) wherein said notch means are respectively located which are spaced apart a distance less than the diameter of the generally circular cross section central passageway.

7. A prosthesis as claimed in any one of claims 1 to 6 wherein said pivot post (143) and said stops (145, 147) project from a pair of protrusions (140) that lie mainly on one side of a diameter of said passageway so that said occluder has a major peripheral section (135) that is generally semicircular and a minor arcuate section (131) that is substantially shorter and that terminates at said pair of opposite straight peripheral segments (137), which in turn terminate in a pair of segments perpendicular thereto which are colinear and interconnect with the ends of said major peripheral section.

8. A prosthesis as claimed in Claim 7 wherein each of said protrusions (140) includes a generally downstream-facing curved surface (150) adjacent which one of said perpendicular segments moves during opening and closing movement of said occluder (125).

9. A prosthesis as claimed in any one of claims 1 to 4 wherein said occluder means comprises a pair of generally semicircular or semielliptical discs (25), each of which has a periphery which includes a straight edge section (31), a major arcuate edge section (35), and a pair of generally diametrically opposite straight segments (37) wherein said notch means (39) are respectively located.

10. A heart valve prosthesis as claimed in any one of claims 1 to 9 having a pair of opposed seat means (161, 163) provided on said interior surface of said annular body, each of said seat means having a surface which is generally that of a portion of a frustum of a cone so that when the periphery of said occluder means engages said pair of seat means in the closed position a good seat is achieved therealong.

**Patentansprüche**

1. Herzklappenprothese (21, 121):

mit einem im wesentlichen ringförmigen Körper (23, 123), dessen Innenfläche (27, 127) einen zentralen Durchgang für die Blutströmung bestimmt,

mit an dem diesem Körper gelagerten Verschlußmitteln (25, 125), die die Blutströmung durch den Durchgang in einer Richtung verhindern, in der anderen Richtung jedoch zulassen und an denen (25, 125) an entgegengesetzten Stellen ihres Umfangs fluchtend ausgerichtete Einschnitte (39, 139) ausgebildet sind,

mit einem Paar fluchtender Vorsprünge (43, 143), die von der Innenfläche des Körpers im wesentlichen radial nach innen ragen, einander gegenüberliegende gebogene Seitenflächen besitzen und so bemessen sind, daß sie in den genannten Einschnitten aufgenommen werden können,

sowie mit Anschlagmitteln (45, 47, 145, 147), die von der Innenfläche (27, 127) des Körpers (23, 123) an seitlich der genannten Vorsprünge nach innen ragen,

dadurch gekennzeichnet,

daß die Vorsprünge (43, 143) Schwenkzapfen für die Verschlußeinrichtung (25, 125) bilden,

daß die Anschlagmittel (45, 47, 145, 147) in radialer Richtung weiter nach innen ragen als die Schwenkzapfen (43, 143), und

daß die Anschlagmittel (45, 47, 145, 147) in an die Schwenkzapfen (43, 143) angrenzenden Bereichen gemeinsam ein Paar gekrümmter Flächen bilden, die jeweils in einem Bereich liegen, der sich zwischen den einander gegenüberliegenden gebogenen Seitenflächen (49a, 49b) der Schwenkzapfen (43, 143) und radial innen von diesen befinden, so daß ein Paar einander gegenüberliegend angeordneter Gelenke gebildet wird, die die Öffnungs- und Schließbewegung der Verschlußmittel (25, 125) abwechselnd unterstützen.

2. Prothese nach Anspruch 1, bei der die Anschlagmittel (45, 47, 145, 147) einstückig mit den Schwenkzapfen (43, 143) ausgebildet sind.

3. Prothese nach Anspruch 2, bei der die gekrümmten Flächen der Anschlagmittel (45, 47, 145, 147) an die gebogenen Seitenflächen (49a, 49b) der Schwenkzapfen (43, 143) angepaßt sind.

4. Prothese nach Anspruch 3, bei der die Anschlagmittel (45, 47, 145, 147) und die Schwenkzapfen (43, 143) einstückig mit dem ringförmigen Körper (23, 123) ausgebildet sind.

5. Prothese nach einem der Ansprüche 1 bis 4, bei der der Brennpunkt jeder der genannten gebogenen Flächen (49a, 49b, 149a, 149b) der Schwenkzapfen (43, 143) jenseits des Mittelpunkts der Schwenkzapfen (43, 143) liegt.

6. Prothese nach einem der Ansprüche 1 bis 5, bei der die Verschlußmittel (25, 125) aus einer einzigen Scheibe (125) bestehen, die ein Paar auf entgegengesetzten Seiten angeordneter geradliniger Abschnitte (137) aufweist, in denen jeweils einer der einschnitte angebracht ist, wobei diese einen Abstand haben, der kleiner ist als der Durchmesser des im wesentlichen kreisförmigen Querschnitts des zentralen Durchgangs.

7. Prothese nach einem der Ansprüche 1 bis 6, bei der der Schwenkzapfen (143) und die Anschläge (145, 147) auf einem Paar von Vorsprüngen (140) herausragen, die vorwiegend auf einer Seite eines Durchmesser des Durchgangs liegen, so daß das Verschlußteil einen im wesentlichen halbkreisförmigen Umfangsabschnitt (135) besitzt und einen kleineren bogenförmigen Umfangsabschnitt (131), der merklich kürzer ist und in dem genannten Paar von einander gegenüberliegenden geradlinigen Umfangsabschnitten (137) endet, die ihrerseits in einem Paar von senkrecht zu ihnen verlaufenden Abschnitten enden, die auf einer Linie liegen und mit den Enden des größeren Umfangsabschnitts in Verbindung stehen.

8. Prothese nach Anspruch 7, bei der jeder der genannten Vorsprünge (140) eine in stromabwärtiger Richtung weisende gebogene Fläche (150) aufweist, an der sich einer der senkrecht verlaufenden Abschnitte während der Öffnungs- und Schließbewegung des Verschlußteils (125) vorbeibewegt.

9. Prothese nach einem der Ansprüche 1 bis 4, bei der das Verschlußteil aus zwei im wesentlichen halbkreisförmigen oder halbeliptischen Scheiben (125) besteht, deren Umfangslinie jeweils einen geradlinigen Kantenabschnitt (31), einen größeren bogenförmigen Kantenabschnitt (35) und zwei einander diametral gegenüberliegende geradlinige Abschnitte (37) aufweist, in denen jeweils einer der Einschnitte (39) angeordnet ist.

10. Prothese nach einem der Ansprüche 1 bis 9, mit zwei auf der Innenfläche des ringförmigen Körpers ausgebildeten Sitzen (161, 163), deren Oberfläche jeweils im wesentlichen einem Teil eines Kegelstumpfs entspricht, so daß dann, wenn das Verschlußteil in Schließstellung mit seinem Umfang an diesen beiden Sitzen zum Anschlag kommt, eine gute Anlage entlang derselben gewährleistet ist.

**Revendications**

1. Prothèse de valvule du coeur (21, 121) comprenant un corps (23, 123) globalement annulaire ayant une surface intérieure (27, 127) qui définit un passage central pour le flux sanguin y passant, des moyens de clapet (25, 125) supportés sur ledit corps et bloquant l'écoulement de sang dans un sens mais permettant l'écoulement du sang par ledit passage dans l'autre sens, lesdits moyens de clapet (25, 125) étant formés avec des moyens encoches (39, 139) alignés en des endroits opposés sur la périphérique de ceux-ci, une paire de saillies (43, 143) alignées se projetant globalement radialement vers l'intérieur à partir de ladite surface intérieure dudit corps, les saillies ayant des faces latérales opposées arquées et étant d'une taille adaptée pour s'engager à l'intérieur desdits moyens encoches, et des moyens de butée (45, 47, 146, 147) se projetant vers l'intérieur à partir de ladite surface intérieure à des endroits globalement adjacents à chacune desdites saillies, caractérisée en ce que lesdites saillies (43, 143) sont des pions pivots pour lesdits moyens de fermeture (25, 125) et que lesdits moyens de butée (45, 47, 145, 147) s'étendent radialement plus loin vers l'intérieur que lesdits pions pivots (43, 143), lesdits moyens de butée (45, 47, 145, 147) ensemble présentent une paire de surfaces courbes (55, 57, 155, 157) dans des régions adjacentes auxdits pions pivots (43, 143), chacune des surfaces courbes étant située à un endroit intermédiaire entre lesdites surfaces latérales arquées (49a, 49b) et lesdits pions pivots (43, 143) et radialement vers l'intérieur, de façon à fournir une paire de points d'appui en regard qui alternativement aident à définir le mouvement d'ouverture et de fermeture desdits moyens de clapet (25, 125).

2. Prothèse selon la revendication 1, dans laquelle lesdits moyens de butée (45, 47, 145, 147) sont d'un seul tenant avec lesdits pions pivots (43, 143).

3. Prothèse selon la revendication 2, dans laquelle les surfaces courbes desdits moyens de butée (45, 47, 145, 147) se fondent avec lesdites surfaces latérales arquées (49a, 49b) des pions pivots.

4. Prothèse selon la revendication 3, dans laquelle lesdits moyens de butée (45, 47, 145, 147) et les pions pivots (43, 143) sont d'un seul tenant avec ledit corps annulaire (23, 123).

5. Prothèse selon l'une quelconque des revendications 1 à 4 dans laquelle le point focal de chacune desdites surfaces arquées (49a, 49b, 149a, 149b) desdits pions pivots (43, 143) est situé au-delà du point de centrage desdits pions pivots (43, 143).

6. Prothèse selon l'une quelconque des revendications 1 à 5, dans laquelle lesdits moyens de clapet (25, 125) sont un disque unique (125) ayant une paire de segments droits (137) opposés à la périphérie, dan lesquels se situent respectivement lesdits moyens encoches et qui sont écartés d'une longueur inférieure au diamètre du passage central de section transversale globalement circulaire.

7. Prothèse selon l'une quelconque des revendications 1 à 6, dans laquelle ledit pion pivot (143) et lesdites butées (145, 147) se projettent à partir d'une paire de protubérances (140) disposées principalement sur un côté d'un diamètre dudit passage, de sorte que ledit clapet a une section périphérique majeure (135) globalement semi-circulaire et une section mineure arquée (131) sensiblement plus courte et se terminant à ladite paire de segments droits (137) opposés qui euxmêmes se terminent par une paire de segments perpendiculaires à ceux là, qui sont colinéaires et qui se raccordent avec les extrémités de ladite section périphérique majeure.

8. Prothèse selon la revendication 7, dans laquelle chacune desdites protubérances comporte une surface courbe (150) regardant l'aval, adjacente à celui desdits segments perpendiculaires qui est en mouvement pendant le mouvement d'ouverture et de fermeture du dit clapet (125).

9. Prothèse selon l'une quelconque des revendications 1 à 4 dans laquelle lesdits moyens de clapet comprennent une paire de disques (25) globalement semi-circulaires ou semi-elliptiques, chacun d'entre-eux ayant un contour comportant une section à bord droit, une section majeure à bord arqué, et une paire de section globalement droites (37) globalement diamétralement opposées dans lesquelles sont respectivement ménagés lesdits moyens encoches (39).

10. Prothèse de valvule du coeur selon l'une quelconque des revendications 1 à 9, ayant une paire de moyen de siège ménagés sur ladite surface intérieure dudit corps annulaire, chacun de ces moyens de siège ayant une surface qui est globalemnnt celle d'une partie d'un tronc de cône de manière que lorsque le contour desdits moyens de clapet est en contact avec ladite paire de moyens de siège dans la position de fermeture, un bon repos est obtenu tout le long de ceux-ci.

Fig.1.

Fig.2.

Fig.3.

Fig.4.

Fig.5.

Fig.6.

Fig.7.

Fig.8.

Fig.9
Fig.10
Fig.11
Fig.12
Fig.13
Fig.14
Fig.15